# FASCICULE DE BREVET EUROPEEN

(11) **EP 3 283 216 B1**
(45) Date de publication et mention de la délivrance du brevet: **01.01.2020**
(21) Numéro de dépôt: 16707672.8
(22) Date de dépôt: 22.02.2016
(51) Int. Cl.: B01J 21/02, B01J 21/00, B01J 23/00, B01J 23/74, B01J 23/75, B01J 21/04, B01J 21/08, C07C 1/04, C10G 2/00, B01J 23/46, B01J 37/02, B01J 35/10

(54) **CATALYSEUR COMPRENANT UNE PHASE ACTIVE DOPEE AU BORE**
KATALYSATOR MIT EINER BOR-DOTIERTEN PHASE
CATALYST COMPRISING A BORON DOPED PHASE

(30) Priorité: 16.04.2015 FR 1553399
(43) Date de publication de la demande: 21.02.2018
(73) Titulaire: IFP Energies nouvelles, 92500 Rueil-Malmaison (FR)
(72) Inventeur: CHENEVIER, Romain, 07340 Peaugres (FR); DECOTTIGNIES, Dominique, 69230 Saint-Genis-Laval (FR); LECOCQ, Vincent, 69530 Orlienas (FR); VELLY, Marie, 42520 Maclas (FR)
(74) Mandataire: IFP Energies nouvelles
(86) Numéro de dépôt international: PCT/EP2016/053631
(87) Numéro de publication internationale: WO 2016/165859

(56) Documents cités:
- EP-A1- 2 921 227
- FR-A1- 2 879 478
- US-A1- 2004 054 016

## Description

### Domaine technique

La présente invention concerne le domaine des catalyseurs utilisés pour les réactions de synthèse d'hydrocarbures à partir d'un mélange de gaz comprenant du monoxyde de carbone et de l'hydrogène, généralement appelée synthèse Fischer-Tropsch.

### Etat de la technique

Les procédés de synthèse Fischer-Tropsch permettent d'obtenir une large gamme de coupes hydrocarbonées à partir du mélange CO + H₂, communément appelé gaz de synthèse. L'équation globale de la synthèse Fischer-Tropsch peut s'écrire de la manière suivante :

n CO + (2n+1) H₂ → CₙH₂ₙ₊₂ + n H₂O

La synthèse Fischer-Tropsch est au cœur des procédés de conversion du gaz naturel, du charbon ou de la biomasse en carburants ou en intermédiaires pour l'industrie chimique. Ces procédés sont appelés GTL (« Gas to Liquids » selon la terminologie anglo-saxonne) dans le cas de l'utilisation de gaz naturel comme charge initiale, CTL (« Coal to Liquids » selon la terminologie anglo-saxonne) pour le charbon, et BTL (« Biomass to Liquids » selon la terminologie anglo-saxonne) pour la biomasse.

Dans chacun de ces cas, la charge initiale est tout d'abord gazéifiée en un gaz de synthèse qui comprend un mélange de monoxyde de carbone et de dihydrogène. Le gaz de synthèse est ensuite transformé majoritairement en paraffines grâce à la synthèse Fischer-Tropsch, et ces paraffines peuvent ensuite être transformées en carburants par un procédé d'hydroisomérisation-hydrocraquage. Par exemple, des procédés de transformation tels que l'hydrocraquage, le déparaffinage, et l'hydroisomérisation des coupes lourdes (C16+) permettent de produire différents types de carburants dans la gamme des distillats moyens: gazole (coupe 180-370°C) et kérosène (coupe 140-300°C). Les fractions plus légères C5-C15 peuvent être distillées et utilisées comme solvants.

La réaction de synthèse Fischer-Tropsch peut être réalisée dans différents types de réacteurs (lit fixe, mobile, ou triphasique (gaz, liquide, solide) par exemple de type autoclave parfaitement agité, ou colonne à bulles), et les produits de la réaction présentent notamment la caractéristique d'être exempts de composés soufrés, azotés ou de type aromatique.

Dans une mise en œuvre dans un réacteur de type colonne à bulles (ou "slurry bubble column" selon la terminologie anglaise, ou encore "slurry" dans une expression simplifiée), qui met en œuvre un catalyseur divisé à l'état de poudre très fines, typiquement de l'ordre de quelques dizaines de micromètres, cette poudre formant une suspension avec le milieu réactionnel.

La réaction Fischer-Tropsch se déroule de manière classique entre 1 et 4 MPa (10 et 40 bars), à des températures comprises traditionnellement entre 200°C et 350°C. La réaction est globalement exothermique, ce qui nécessite une attention particulière à la mise en œuvre du catalyseur.

Les catalyseurs employés pour la synthèse Fischer-Tropsch sont essentiellement des catalyseurs à base de cobalt ou de fer, même si d'autres métaux peuvent être utilisés. Néanmoins, le cobalt et le fer offrent un bon compromis performances/prix par rapport aux autres métaux.

Les méthodes conventionnelles de préparation des catalyseurs métalliques supportés utilisés pour la synthèse Fischer-Tropsch consistent à déposer un sel métallique ou un complexe de coordination métal-ligand sur le support, puis à réaliser un ou plusieurs traitement(s) thermique(s) réalisé(s) sous air, suivi d'un traitement réducteur effectué ex-situ ou in-situ.

Afin d'améliorer les propriétés catalytiques des catalyseurs métalliques supportés utilisés pour la synthèse Fischer-Tropsch, des éléments dopants peuvent être ajoutés soit dans la phase active des catalyseurs ou soit directement dans le support. L'élément dopant permet en général d'améliorer la réductibilité du métal du groupe VIIIB, et donc son activité, ou sa sélectivité, ou encore de ralentir sa désactivation. Le bore rentre dans la catégorie des éléments dopants de la phase active des catalyseurs.

Le document US 6,593,378 décrit un procédé de synthèse Fischer-Tropsch utilisant un catalyseur comprenant du cobalt en tant que phase active et un support préparé à partir d'un support alumine et de borate d'aluminium. Le document démontre que la présence de borate d'aluminium dans le support permet d'améliorer l'activité du catalyseur.

L'article paru dans Journal of Catalysis, 2011, 280, 50-59 par Sayes et al. décrit un catalyseur comprenant une phase active comprenant 20% en poids de cobalt et entre 0,5 % à 2,0 % en poids de bore par rapport au poids total du catalyseur, et un support en alumine (γ-Al203). Le bore est approvisionné, seul, lors de la dernière étape de préparation du catalyseur. Cependant, l'ajout entre 0,5 % et 2 % en poids de bore a un effet limité sur la réductibilité du cobalt et sur les performances du catalyseur. Il permet en revanche d'améliorer de manière significative la stabilité du catalyseur, en limitant la formation de dépôt carboné sur le catalyseur.

Le document WO02/068564 décrit un catalyseur comprenant une phase active comprenant 20% en poids de cobalt et 0,5 % en poids de bore par rapport au poids total du catalyseur, et un support en alumine. Le procédé de préparation de ce catalyseur comprend une étape dans laquelle le précurseur de cobalt et le précurseur de bore sont ajoutés simultanément (co-imprégnation). Cependant, l'ajout du bore à une telle teneur dans le catalyseur ne permet pas d'améliorer significativement son activité.

La Demanderesse a découvert de manière surprenante qu'un catalyseur contenant une phase active comprenant au moins un métal du groupe VIIIB et du bore, dont la teneur en bore est inférieure strictement à 0,5% en poids par rapport au poids total du catalyseur, déposée sur un support contenant de la silice, de l'alumine et une spinelle permet d'améliorer leurs performances catalytiques dans un procédé de type Fischer-Tropsch, et plus particulièrement permet d'augmenter de manière significative l'activité dudit catalyseur. Un tel catalyseur est obtenu par un procédé de préparation comprenant au moins une étape dans laquelle le précurseur d'au moins un métal du groupe VIIIB et un précurseur de bore sont ajoutés simultanément en solution. Le procédé Fischer-Tropsch est alors effectué en présence d'un catalyseur ayant une activité améliorée, sans pour autant diminuer sa stabilité et le taux de sélectivité.

### Objets de l'invention

Un premier objet de l'invention concerne un catalyseur contenant une phase active comprenant au moins un métal du groupe VIIIB choisi parmi le cobalt, le nickel, le ruthénium et le fer déposé sur un support contenant de la silice, de l'alumine, et au moins une spinelle simple MAI₂O₄ ou mixte MₓM'₍₁₋ₓ₎Al₂₀O₄ partielle ou non, où M et M' sont des métaux distincts choisis dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn) et où x est compris entre 0 et 1, les valeurs 0 et 1 étant elles-mêmes exclues, caractérisé en ce que ladite phase active comprend en outre du bore, la teneur en bore étant comprise entre 0,02 % et 0,2 % en poids par rapport au poids total du catalyseur.

Avantageusement, ledit support est une silice-alumine dans laquelle la spinelle est incluse. De préférence, la teneur en spinelle est comprise entre 3 et 50 % poids par rapport au poids du support.

De préférence, la teneur en métal M ou M' est comprise entre 1 et 20 % poids par rapport au poids du support.

Avantageusement, M est le cobalt dans le cas d'une spinelle simple, et M est le cobalt et M' est le magnésium ou le zinc dans le cas d'une spinelle mixte.

De préférence, la teneur en métal du groupe VIIIB de la phase active est comprise entre 0,01 et 60 % poids par rapport au poids du catalyseur.

Un autre objet concerne un procédé de préparation d'un catalyseur selon l'invention, lequel procédé comprenant au moins une étape dans laquelle on imprègne ledit support contenant de la silice, de l'alumine et la spinelle par une solution aqueuse ou organique comprenant au moins un sel de métal du groupe VIIIB choisi parmi le cobalt, le nickel, le ruthénium et le fer, et au moins un précurseur de bore.

Avantageusement, le procédé selon l'invention comprend les étapes suivantes :
on fournit un support contenant de la silice et de l'alumine ;
on imprègne ledit support par une solution aqueuse ou organique comprenant au moins un sel de métal M ou M' choisi dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn), puis on sèche et on calcine à une température entre 700 et 1200°C, de manière à obtenir une spinelle simple MAI₂O₄ ou mixte MₓM'₍₁₋ₓ)Al₂O₄ partielle ou non, où M et M' sont des métaux distincts et où x est compris entre 0 et 1, les valeurs 0 et 1 étant elles-mêmes exclues ;
on imprègne une première fois ledit support contenant de la silice, de l'alumine et la spinelle par une solution aqueuse ou organique comprenant au moins un sel de métal du groupe VIIIB choisi parmi le cobalt, le nickel, le ruthénium et le fer ;
on imprègne une deuxième fois ledit support contenant de la silice, de l'alumine et la spinelle par une solution aqueuse ou organique comprenant au moins un sel de métal du groupe VIIIB choisi parmi le cobalt, le nickel, le ruthénium et le fer ;
procédé dans lequel ladite solution aqueuse ou organique imprégnée à l'étape c) et/ou d) comprend en outre le précurseur de bore.

De préférence, le précurseur de bore est introduite uniquement dans la solution aqueuse ou organique approvisionné à l'étape d).

Avantageusement, la teneur la teneur du ou des métaux du groupe VIIIB approvisionnée à l'étape c) est comprise entre 0,005 et 30 % poids par rapport au poids du catalyseur.

Avantageusement, la teneur du ou des métaux du groupe VIIIB approvisionnée à l'étape d) est comprise entre 0,005 et 30 % en poids par rapport au poids total du catalyseur.

De préférence, le précurseur de bore est l'acide borique.

Avantageusement, on sèche le catalyseur obtenu à l'étape d) à une température comprise entre 30 minutes et 3 heures, puis on calcine ledit catalyseur séché sous atmosphère oxydante à une température comprise entre 320°C et 460°C.

De préférence, on réduit le catalyseur séché et calciné à une température comprise entre 200°C et 500°C.

Un autre objet concerne un procédé Fischer-Tropsch de synthèse d'hydrocarbures dans lequel le catalyseur selon l'invention ou préparé par le procédé selon l'invention est mis en contact avec une charge comprenant du gaz de synthèse sous une pression totale comprise entre 0,1 et 15 MPa, sous une température comprise entre 150 et 350°C, et à une vitesse volumique horaire comprise entre 100 et 20000 volumes de gaz de synthèse par volume de catalyseur et par heure avec un rapport molaire H₂/CO du gaz de synthèse entre 0,5 et 4.

### Description détaillée de l'invention

Dans la description suivante, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIIIB selon la classification CAS correspond aux métaux des colonnes 8, 9 et 10 selon la nouvelle classification IUPAC.

Les propriétés texturales et structurales du support et du catalyseur décrits ci-après sont déterminées par les méthodes de caractérisation connues de l'homme du métier. Le volume poreux total et la distribution poreuse sont déterminés dans la présente invention par porosimétrie au mercure (cf. Rouquerol F.; Rouquerol J.; Singh K. « Adsorption by Powders & Porous Solids: Principle, methodology and applications », Academic Press, 1999). Plus particulièrement, le volume poreux total est mesuré par porosimétrie au mercure selon la norme ASTM D4284-92 avec un angle de mouillage de 140°, par exemple au moyen d'un appareil modèle Autopore III™ de la marque Micromeritics™. La surface spécifique est déterminée dans la présente invention par la méthode B.E.T, méthode décrite dans le même ouvrage de référence que la porosimétrie au mercure, et plus particulièrement selon la norme ASTM D3663-03.

### Catalyseur

L'invention concerne un catalyseur contenant une phase active comprenant au moins un métal du groupe VIIIB déposée sur un support contenant de la silice, de l'alumine et au moins une spinelle simple MAl₂O₄ ou mixte MₓM'₍₁₋ₓ₎Al₂O₄ partielle ou non, où M et M' sont des métaux distincts choisis dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn) et où x est compris entre 0 et 1, les valeurs 0 et 1 étant elles-mêmes exclues, la phase active dudit catalyseur comprenant en outre du bore, la teneur en bore étant comprise entre 0,02 % et 0,2 % en poids par rapport au poids total du catalyseur.

La teneur en métal du groupe VIIIB de la phase active choisi parmi le cobalt, le nickel, le ruthénium et le fer est entre 0,01 et 60 % poids par rapport au poids du catalyseur.

Dans le cas où la phase active comprend au moins un métal choisi parmi le cobalt, le nickel et le fer, la teneur en ledit métal représente de 1 à 60 % poids, de préférence de 5 à 30 % poids, et de manière très préférée de 10 à 30 % poids par rapport au poids du catalyseur.

Dans le cas où la phase active comprend du ruthénium, la teneur en ruthénium est comprise entre 0,01 et 10 % poids, et de manière préférée entre 0,05 et 5 % poids par rapport au poids du catalyseur.

La teneur en bore est comprise entre 0,02 et 0,2 % en poids par rapport au poids total du catalyseur.

La phase active dudit catalyseur peut avantageusement comprendre en outre au moins un élément dopant supplémentaire choisi parmi un métal noble des groupes VIIB ou VIIIB. L'élément dopant supplémentaire permet d'améliorer la réductibilité du métal du groupe VIIIB, et donc son activité, ou sa sélectivité, ou encore de ralentir sa désactivation. Lorsqu'au moins un dopant supplémentaire est présent, la teneur en dopant(s) supplémentaire(s) est généralement comprise entre 20 ppm et 1 % poids, et de préférence entre 0,01 à 0,5 poids par rapport au poids du catalyseur. Dans le cas où le dopant est choisi parmi un métal noble des groupes VIIB ou VIIIB, il est de préférence choisi parmi le platine (Pt), le palladium (Pd), le rhodium (Rh) ou encore le rhénium (Re). Plus préférentiellement, le dopant est le platine (Pt).

Ladite phase active comprend donc du bore et au moins un métal du groupe VIIIB choisi parmi le cobalt, le nickel, le ruthénium et le fer, et éventuellement un élément dopant supplémentaire. De manière préférée, ladite phase active comprend du cobalt, du bore et du platine. De manière préférée, ladite phase active est constituée de cobalt et de bore. De manière encore plus préférée, ladite phase active est constituée de cobalt, de bore et de platine.

Le support dudit catalyseur employé pour la mise en œuvre du procédé de synthèse d'hydrocarbures selon l'invention est un support d'oxydes contenant de l'alumine, de la silice, et au moins une spinelle telle que décrite ci-dessus.

L'alumine présente dans le support d'oxydes présente généralement une structure cristallographique du type alumine delta (δ), gamma (γ), thêta (θ) ou alpha (α), seule ou en mélange.

Le support contenant de l'alumine, de la silice, au moins une spinelle telle que décrite ci-dessus peut être préparé à partir d'alumine quelle que soit sa surface spécifique et la nature de sa répartition poreuse. La surface spécifique de l'alumine à partir de laquelle le support est préparé est généralement comprise entre 50 m²/g et 500 m²/ g, de préférence entre 100 m²/g et 300 m²/g, de façon plus préférée entre 150 m²/g et 250 m²/g. Le volume poreux total de l'alumine à partir de laquelle le support est préparé est généralement compris entre 0,4 ml/g et 1,2 ml/g, et de préférence compris entre 0,45 ml/g et 1 ml/g.

La distribution poreuse des pores dans l'alumine à partir de laquelle le support est préparé peut être de type monomodale, bimodale ou plurimodale. De préférence, elle est de type monomodale. La taille de pores est de l'ordre de 2 à 50 nm, avec une taille moyenne des pores entre 5 et 25 nm, de préférence entre 8 et 20 nm.

Les caractéristiques de l'alumine mentionnées ci-dessus correspondent aux caractéristiques de l'alumine à partir de laquelle le support est préparé, c'est-à-dire avant l'introduction de la silice, des métaux M et éventuellement M' pour la formation de la phase spinelle, de la phase active.

La teneur en silice dans le support varie de 0,5 % poids à 30 % poids, de manière préférée de 1 % poids à 25 % poids, et de manière encore plus préférée de 1,5 à 20 % poids par rapport au poids du support.

On entend par un support contenant de l'alumine et de la silice un support dans lequel le silicium et l'aluminium sont sous forme d'agglomérats de silice ou d'alumine respectivement, d'aluminosilicate amorphe ou toute autre phase mixte contenant du silicium et de l'aluminium. De préférence, l'alumine et la silice sont présents sous forme de mélange d'oxydes SiO₂-Al₂O₃ dénommée silice-alumine. On entend par silice-alumine une alumine comprenant un pourcentage de silice strictement supérieur à 10 % poids allant jusqu'à 30 % poids par rapport au poids du support. Ladite silice-alumine est homogène à l'échelle du micromètre, et de manière encore plus préférée, homogène à l'échelle du nanomètre.

La spinelle présente dans le support d'oxydes est une spinelle simple MAl₂O₄ ou mixte MₓM'_{(1.-x)}Al₂O₄ partielle ou non, où M et M' sont des métaux distincts choisis dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn) et où x est compris entre 0 et 1, les valeurs 0 et 1 étant elles-mêmes exclues.

L'utilisation de phases de structures spinelles du type MAl₂O₄ ou spinelles mixtes MₓM'₍₁₋ₓ₎Al₂O₄ a été décrite dans les documents FR2879478 et WO 2005/072866, M et M' étant généralement des métaux divalents tels que Mg, Sn, Ni, Co, Cu. On peut également citer les publications de Rotan et coll. dans Journal of the European Ceramic Society 33 (2013) 1-6 et Rytter et coll. dans Top. Catal. 54 (2011) 801-810. Dans ce cas, le métal divalent (notamment le nickel) est introduit sous forme d'un précurseur de type nitrate par exemple à hauteur de quelques pourcents sur le support initial contenant de l'alumine. Par calcination à très haute température, la phase spinelle est formée et stabilise l'ensemble du support.

De manière très préférée, M est le cobalt ou le nickel dans le cas d'un spinelle simple. De manière très préférée, M est le cobalt et M' est le magnésium ou le zinc dans le cas d'un spinelle mixte.

De manière particulièrement préférée, la spinelle est une spinelle simple MAl₂O₄ dans laquelle M est le cobalt.

La teneur de la spinelle est généralement entre 3 et 50 % poids, et de manière préférée entre 5 et 40 % poids par rapport au poids du support.

La teneur en métal M ou M' est comprise entre 1 et 20 % poids, et de manière préférée entre 2 et 10 % poids par rapport au poids du support.

La formation de la structure spinelle simple ou mixte dans ledit support, souvent appelé étape de stabilisation du support, peut être effectuée par toute méthode connue de l'Homme du métier. Elle est généralement effectuée en introduisant le métal M ou M' sous forme d'un précurseur de sel par exemple de type nitrate sur le support initial contenant l'alumine. Par calcination à très haute température, la phase spinelle, dans laquelle le métal M ou M' sont sous forme d'aluminate, est formée et stabilise l'ensemble du support.

La présence de phase spinelle dans le catalyseur utilisé dans le procédé Fischer-Tropsch selon l'invention se mesure par réduction en température programmée RTP (ou TPR pour "temperature programmed réduction" selon la terminologie anglo-saxonne) tel que par exemple décrit dans Oil & Gas Science and Technology, Rev. IFP, Vol. 64 (2009), No. 1, pp. 11-12. Selon cette technique, le catalyseur est chauffé sous flux d'un réducteur, par exemple sous flux de dihydrogène. La mesure du dihydrogène consommé en fonction de la température donne des informations quantitatives sur la réductibilité des espèces présentes. La présence d'une phase spinelle dans le catalyseur se manifeste ainsi par une consommation de dihydrogène à une température supérieure à environ 800°C.

De préférence, le support d'oxydes contenant de l'alumine, de la silice, au moins une spinelle telle que décrite ci-dessus est une silice-alumine dans laquelle la spinelle est incluse, ledit support ayant de préférence une teneur en silice entre 0,5% poids à 30% poids par rapport au poids du support, ledit support contenant en plus au moins une spinelle telle que décrite ci-dessus. De préférence, la teneur en silice est supérieure à 10% poids allant jusqu'à 30% poids par rapport au poids du support, ledit support contenant en outre au moins une spinelle telle que décrite ci-dessus.

La surface spécifique du support d'oxydes contenant de l'alumine, de la silice, et au moins une spinelle telle que décrite ci-dessus est généralement comprise entre 50 m²/g et 500 m²/g, de préférence entre 100 m²/g et 300 m²/g, de façon plus préférée entre 150 m²/g et 250 m²/g. Le volume poreux dudit support est généralement compris entre 0,3 ml/g et 1,2 ml/g, et de préférence compris entre 0,4 ml/g et 1 ml/g.

Le support sur lequel est déposée ladite phase active peut présenter une morphologie sous forme de billes, d'extrudés (par exemple de forme trilobes ou quadrilobes) ou de pastilles, notamment lorsque ledit catalyseur est mis en œuvre dans un réacteur fonctionnant en lit fixe, ou présenter une morphologie sous forme de poudre de granulométrie variable, notamment lorsque ledit catalyseur est mis en œuvre dans un réacteur de type colonne à bulles.

La surface spécifique du catalyseur contenant la phase active et le support d'oxydes contenant de l'alumine, de la silice, et au moins une spinelle telle que décrite ci-dessus est généralement comprise entre 50 m²/g et 500 m²/g, de préférence entre 80 m²/g et 250 m²/g, de façon plus préférée entre 90 m²/g et 150 m /g. Le volume poreux dudit catalyseur est généralement compris entre 0,2 ml/g et 1 ml/g, et de préférence compris entre 0,25 ml/g et 0,8 ml/g. De préférence, la distribution poreuse est monomodale.

De manière préférée, le catalyseur selon l'invention contient une phase active comprenant du cobalt et du bore, et éventuellement un élément dopant supplémentaire, de préférence le platine, la teneur en bore étant comprise entre 0,02 et 0,2 % en poids, et un support en silice-alumine dans laquelle une spinelle est incluse, la teneur en silice du support étant de préférence entre 1,5 et 20 % poids par rapport au poids du support, ladite spinelle étant une spinelle simple MAl₂O₄ ou mixte MₓM'₍₁₋ₓ₎Al2O₄ partielle ou non, où M et M' sont des métaux distincts choisis dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn) et où x est compris entre 0 et 1, les valeurs 0 et 1 étant elles-mêmes exclues.

De manière particulièrement préférée, le catalyseur employé pour la mise en œuvre du procédé Fischer-Tropsch selon l'invention est un catalyseur dans lequel la phase active est constituée de cobalt et de bore, et éventuellement du platine, et le support d'oxydes est constitué d'une silice-alumine dans laquelle une spinelle est incluse, la teneur en silice du support est comprise entre 1,5 % et 20 % poids par rapport au poids du support, ladite spinelle étant du CoAl₂O₄, la teneur en bore étant comprise entre 0,001 et 0,5 % en poids par rapport au poids total du catalyseur, la valeur 0,5 étant elle-même exclue. De préférence, la teneur en bore est comprise entre 0,01 et 0,4 % en poids par rapport au poids total du catalyseur, plus préférentiellement entre 0,02 et 0,35 %, et encore plus préférentiellement entre 0,02 et 0,2 % en poids.

### Procédé de préparation du catalyseur

La préparation du catalyseur comporte généralement dans un premier temps la préparation du support d'oxydes contenant de l'alumine, de la silice, au moins une spinelle, puis, dans un deuxième temps l'introduction de la phase active.

Selon l'invention, le procédé de préparation du catalyseur comprend au moins une étape dans laquelle on imprègne ledit support contenant de la silice, de l'alumine et la spinelle par une solution aqueuse ou organique comprenant au moins un sel de métal du groupe VIIIB choisi parmi le cobalt, le nickel, le ruthénium et le fer, et au moins un précurseur de bore (co-imprégnation).

De préférence, l'imprégnation du précurseur du métal du groupe VIIIB choisi parmi le cobalt, le nickel, le ruthénium et le fer peut être réalisée en plusieurs étapes, de préférence en deux étapes. Entre chacune des étapes d'imprégnation, il est préféré d'effectuer éventuellement au moins une étape de séchage et/ou de calcination dans les conditions décrites ci-dessous, et/ou de réduction dans les conditions décrites ci-dessous.

Plus particulièrement, le procédé de préparation du catalyseur selon l'invention comprend les étapes suivantes :
on fournit un support d'oxydes contenant de l'alumine et de la silice ;
on imprègne le support contenant de l'alumine et de la silice par une solution aqueuse ou organique comprenant au moins un sel de métal M ou M' choisi dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn), puis on sèche et on calcine à une température entre 700 et 1200°C, de manière à obtenir une spinelle simple MAl₂O₄ ou mixte MₓM'₍₁₋ₓ₎Al₂O₄ partielle ou non, où M et M' sont des métaux distincts et où x est compris entre 0 et 1, les valeurs 0 et 1 étant elles-mêmes exclues ;
on imprègne une première fois le support d'oxydes contenant de l'alumine, de la silice, et une spinelle par une solution aqueuse ou organique comprenant au moins un sel de métal du groupe VIIIB choisi parmi le cobalt, le nickel, le ruthénium et le fer ;
on imprègne une seconde fois le support d'oxydes contenant de l'alumine, de la silice, et une spinelle par une solution aqueuse ou organique comprenant au moins un sel de métal du groupe VIIIB choisi parmi le cobalt, le nickel, le ruthénium et le fer ;
et la solution aqueuse ou organique imprégnée à l'étape c) et/ou d) comprend un précurseur de bore.

Selon l'étape a), on fournit un support contenant de l'alumine et de la silice. La teneur en silice SiO₂ peut varier de 0,5 % poids à 30 % poids, de manière préférée de 1 % poids à 30% poids, et de manière encore plus préférée de 1,5 à 20 % poids par rapport au poids du support. De préférence, on fournit un support de silice-alumine. Un tel support peut être acheté ou fabriqué, par exemple par atomisation d'un précurseur d'alumine en présence d'un composé comprenant du silicium. Le support contenant de l'alumine et de la silice peut être préparé par tout autre moyen connu de l'Homme de l'Art, par exemple par imprégnation d'un composé organosilylé de type TEOS (tetraethylorthosilicate) sur une alumine. Dans ce cas, cette imprégnation, suivie d'un séchage et d'une calcination, est préliminaire à l'étape a) décrite ci-dessus.

Le solide contenant de l'alumine, de la silice peut ensuite être séché et calciné. Le séchage est avantageusement effectué à une température comprise entre 60°C et 200°C, de préférence pendant une durée allant de 30 minutes à trois heures. La calcination est avantageusement effectuée à une température comprise entre 200°C et 1100°C, de préférence pendant une durée allant de 1 heure à 24 heures, et de manière préférée de 2 heures à 8 heures. La calcination est généralement effectuée sous atmosphère oxydante, par exemple sous air, ou sous air appauvri en oxygène ; elle peut également être effectuée au moins en partie sous azote.

Toutes les étapes de séchage et de calcination décrites dans la présente description peuvent être réalisées par toute technique connue de l'Homme de l'Art : lit fixe, lit fluidisé, étuve, four à moufles, four tournant.

L'étape b), consiste en l'imprégnation, de préférence à sec, dudit support contenant de l'alumine, de la silice, par une solution aqueuse d'un ou plusieurs sels d'un métal M ou M' choisis dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium K, le fer (Fe) et le manganèse (Mn), de préférence le cobalt, le nickel, le magnésium, le calcium et le zinc et de manière très préférée le cobalt et le nickel, et de manière particulièrement préférée le cobalt, suivie d'un séchage et d'une calcination à une température comprise entre 700 et 1200°C.

Le métal M ou M' est mis au contact du support par l'intermédiaire de tout précurseur métallique soluble en phase aqueuse. De manière préférée, lorsque le métal M ou M' appartient au groupe VIIIB, alors le précurseur du métal du groupe VIIIB est introduit en solution aqueuse, de préférence sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. Dans le cas préféré où le métal M est le cobalt, le précurseur de cobalt avantageusement utilisé est le nitrate de cobalt, l'oxalate de cobalt ou l'acétate de cobalt.

La teneur en métal M ou M' est avantageusement comprise entre 1 et 20 % poids et de préférence entre 2 et 10 % poids par rapport à la masse totale du support final.

Le séchage est avantageusement effectué à une température comprise entre 60°C et 200°C, de préférence pendant une durée allant de 30 minutes à trois heures.

La calcination est effectuée à une température comprise entre 700 et 1200°C, de préférence comprise entre 850 et 1200°C, et de manière préférée comprise entre 850 et 900°C, généralement pendant une durée comprise entre une heure et 24 heures et de préférence comprise entre 2 heures et 5 heures. La calcination est généralement effectuée sous atmosphère oxydante, par exemple sous air, ou sous air appauvri en oxygène ; elle peut également être effectuée au moins en partie sous azote. Elle permet de transformer les précurseurs M et M' et l'alumine en structure de type spinelle (aluminate de M et M').

Selon une variante, la calcination peut également être effectuée en deux étapes, ladite calcination est avantageusement réalisée à une température comprise entre 300°C et 600°C sous air pendant une durée comprise entre une demi-heure et trois heures, puis à une température comprise entre 700°C et 1200°C, de préférence comprise entre 850 et 1200°C et de manière préférée entre 850 et 900°C, généralement pendant une durée comprise entre une heure et 24 heures, et de préférence comprise entre 2 heures et 5 heures.

Ainsi, à l'issue de ladite étape b), ledit support contenant de l'alumine et de la silice contient en outre au moins une spinelle simple MAl₂O₄ ou mixte MₓM'₍₁₋ₓ₎Al₂O₄ partielle ou non, dans laquelle les métaux M et M' sont sous forme d'aluminates.

De préférence, à l'étape c), on imprègne ledit support avec une solution comprenant uniquement le précurseur du métal du groupe VIIIB choisi parmi le cobalt, le nickel, le ruthénium et le fer ; et on imprègne à l'étape d) ledit support de l'étape c) avec une solution comprenant le précurseur de bore et le précurseur du métal du groupe VIIIB choisi parmi le cobalt, le nickel, le ruthénium et le fer.

En particulier, les étapes c) et d) d'imprégnation sont réalisées par imprégnation à sec, par imprégnation en excès, ou encore par dépôt - précipitation selon des méthodes bien connues de l'Homme du métier. De manière préférée, les étapes c) et d) d'imprégnation sont réalisées par imprégnation à sec, de préférence à température ambiante, et de manière préférée à une température égale à 20°C.

Plus particulièrement, les étapes d'imprégnation c) et/ou d) consistent à mettre en contact ledit support avec une solution comprenant au moins un précurseur dudit métal du groupe VIIIB et une solution comprenant un précurseur de bore (approvisionnée à l'étape c) et/ou d)). Le volume total de solutions imprégnées aux étapes c) et d) est égal au volume poreux dudit support à imprégner.

Le précurseur métallique du ou des métaux du groupe VIIIB est approvisionnée en solution à une concentration voulue pour obtenir sur le catalyseur final la teneur en métal visée, avantageusement une teneur en métal comprise entre 0,01 et 60 % poids, et de préférence entre 5 et 30 % poids par rapport au poids du catalyseur. De préférence, la teneur en métal du ou des métaux du groupe VIIIB approvisionnée à l'étape c) est comprise entre 0,005 et 30 % poids par rapport au poids total du catalyseur, de préférence entre 1 et 30% en poids, et encore plus préférentiellement entre 2,5 et 15 % en poids. La teneur en métal du ou des métaux du groupe VIIIB approvisionnée à l'étape d) est comprise entre 0,005 et 30 % en poids par rapport au poids total du catalyseur, de préférence entre 1 et 30 % en poids, et encore plus préférentiellement entre 2,5 et 15 %.

La solution contenant le précurseur de bore est approvisionnée à une concentration voulue pour obtenir sur le catalyseur final une teneur en bore comprise entre 0,02 et 0,2 % en poids par rapport au poids du catalyseur. De manière préférée, le précurseur de bore est approvisionnée en solution à l'étape d), simultanément avec le précurseur du ou des métaux du groupe VIIIB (co-imprégnation).

Le métal ou les métaux du groupe VIIIB sont mis au contact du support par l'intermédiaire de tout précurseur métallique soluble en phase aqueuse ou en phase organique. Lorsqu'il est introduit en solution organique, le précurseur du métal du groupe VIIIB est de préférence l'oxalate ou l'acétate dudit métal du groupe VIIIB. De manière préférée, le précurseur du métal du groupe VIIIB est introduit en solution aqueuse, de préférence sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. Dans le cas préféré où le métal du groupe VIIIB est le cobalt, le précurseur de cobalt avantageusement utilisé est le nitrate de cobalt, l'oxalate de cobalt ou l'acétate de cobalt. De manière la plus préférée, le précurseur utilisé est le nitrate de cobalt.

La source de bore peut être l'acide borique, de préférence l'acide orthoborique H₃BO₄ le biborate ou le pentaborate d'ammonium, l'oxyde de bore, les esters boriques. Le bore peut par exemple être introduit sous la forme d'un mélange d'acide borique, d'eau oxygénée et un composé organique basique contenant de l'azote tels que l'ammoniaque, les aminés primaires et secondaires, les aminés cycliques, les composés de la famille de la pyridine et des quinoléines et les composés de la famille du pyrrole. Le bore peut être introduit par exemple par une solution d'acide borique dans un mélange eau/alcool.

Lesdites étapes d'imprégnation c) et d) du support avec la phase active, peut également avantageusement comprendre au moins une étape supplémentaire consistant à déposer au moins un élément dopant supplémentaire choisi parmi un métal noble des groupes VIIB ou VIIIB sur ledit support d'oxydes. Le dopant est de préférence choisi parmi le platine (Pt), le palladium (Pd), le rhodium (Rh) ou encore le rhénium (Re). Plus préférentiellement, l'élément dopant supplémentaire est le platine (Pt). Le dépôt du dopant supplémentaire sur le support peut avantageusement être réalisé par toute méthode connue de l'Homme du métier, préférentiellement par imprégnation dudit support d'oxydes par au moins une solution contenant au moins un précurseur dudit dopant supplémentaire, et de préférence, par imprégnation à sec ou par imprégnation en excès. Cette solution contient au moins un précurseur dudit dopant supplémentaire à la concentration voulue pour obtenir sur le catalyseur final la teneur en dopant supplémentaire comprise entre 20 ppm et 11 % poids, et de préférence entre 0,01 à 0,5 % poids par rapport au poids du catalyseur. Par la suite, le catalyseur contenant le dopant est séché et calciné dans les mêmes conditions que celles décrites dans les étapes de séchage et de calcination lors de l'imprégnation de la phase active.

L'introduction de l'élément dopant supplémentaire peut être réalisée en même temps que l'imprégnation de la solution aqueuse ou organique comprenant ledit sel de métal du groupe VIIIB choisi parmi le cobalt, le nickel, le ruthénium et le fer, et ledit précurseur de bore, i.e. à l'étape c) et/ou d), ou lors d'une étape supplémentaire (après l'étape d)). De préférence, l'élément dopant supplémentaire est introduit en même temps que l'imprégnation de ladite solution aqueuse ou organique comprenant ledit sel de métal du groupe VIIIB choisi parmi le cobalt, le nickel, le ruthénium et le fer, et ledit précurseur de bore.

Le catalyseur ainsi obtenu est ensuite séché et calciné. Le séchage est avantageusement effectué à une température comprise entre 60°C et 200°C, de préférence pendant une durée allant de 30 minutes à trois heures. La calcination est avantageusement réalisée à une température comprise entre 320°C et 460°C, de préférence entre 350 et 440°C et de manière préférée entre 360 et 420°C. Elle est préférentiellement réalisée pendant une durée comprise entre 15 min et 15h et de préférence entre 30 min et 12h et de manière encore plus préférée entre 1h et 6h. La calcination est généralement effectuée sous atmosphère oxydante, par exemple sous air, ou sous air appauvri en oxygène ; elle peut également être effectuée au moins en partie sous azote.

Préalablement à son utilisation dans le réacteur catalytique de synthèse Fischer-Tropsch, le catalyseur subit généralement un traitement réducteur, par exemple sous hydrogène pur ou dilué, à haute température, destiné à activer le catalyseur et à former des particules de métal à l'état zéro valent (sous forme métallique). Ce traitement peut être effectué in situ (dans le même réacteur que celui où est opéré la synthèse Fischer-Tropsch), ou ex situ avant d'être chargé dans le réacteur. La température de ce traitement réducteur est préférentiellement comprise entre 200°C et 500°C et sa durée est généralement comprise entre 2 et 20 heures.

### Procédé Fischer-Tropsch

La présente invention concerne également un procédé Fischer-Tropsch par la mise en contact d'une charge comprenant du gaz de synthèse sous des conditions opératoires de synthèse Fischer-Tropsch avec au moins un catalyseur selon l'invention ou préparé selon le procédé de préparation de l'invention.

Le procédé Fischer-Tropsch permet la production d'hydrocarbures essentiellement linéaires et saturés C5+. Conformément à l'invention, on entend par hydrocarbures essentiellement linéaires et saturés C5+, des hydrocarbures dont la proportion en composés hydrocarbonés ayant au moins 5 atomes de carbone par molécule représente au moins 50% en poids, de préférence au moins 80% en poids de l'ensemble des hydrocarbures formés, la teneur totale en composés oléfiniques présents parmi lesdits composés hydrocarbonés ayant au moins 5 atomes de carbone par molécule étant inférieure à 15% poids. Les hydrocarbures produits par le procédé de l'invention sont ainsi des hydrocarbures essentiellement paraffiniques, dont la fraction présentant les points d'ébullition les plus élevés peut être convertie avec un rendement élevé en distillats moyens (coupes gazole et kérosène) par un procédé catalytique d'hydroconversion tel que l'hydrocraquage et/ou l'hydroisomérisation.

De manière préférée, la charge employée pour la mise en œuvre du procédé de l'invention, est constituée par le gaz de synthèse qui est un mélange de monoxyde de carbone et d'hydrogène de rapports molaires H₂/CO pouvant varier entre 0,5 et 4 en fonction du procédé de fabrication dont il est issu. Le rapport molaire H₂/CO du gaz de synthèse est généralement voisin de 3 lorsque le gaz de synthèse est obtenu à partir du procédé de vaporeformage d'hydrocarbures ou d'alcool. Le rapport molaire H₂/CO du gaz de synthèse est de l'ordre de 1,5 à 2 lorsque le gaz de synthèse est obtenu à partir d'un procédé d'oxydation partielle. Le rapport molaire H₂/CO du gaz de synthèse est généralement voisin de 2,5 lorsqu'il est obtenu à partir d'un procédé de reformage autotherme. Le rapport molaire H₂/CO du gaz de synthèse est généralement voisin de 1 lorsqu'il est obtenu à partir d'un procédé de gazéification et de reformage d'hydrocarbures au CO₂ (dit reformage sec).

Le procédé Fischer-Tropsch selon l'invention est opéré sous une pression totale comprise entre 0,1 et 15 MPa, de préférence entre 0,5 et 10 MPa, sous une température comprise entre 150 et 350°C, de préférence entre 180 et 270°C. La vitesse volumique horaire est avantageusement comprise entre 100 et 20000 volumes de gaz de synthèse par volume de catalyseur et par heure (100 à 20000 h⁻¹) et de préférence entre 400 et 10000 volumes de gaz de synthèse par volume de catalyseur et par heure (400 à 10000 h⁻¹). Le procédé Fischer-Tropsch selon l'invention peut être effectué en réacteur de type autoclave parfaitement agité, lit bouillonnant, colonne à bulles, lit fixe ou lit mobile. De préférence, il est effectué dans un réacteur de type colonne à bulles.

De ce fait, la taille des grains du catalyseur utilisé dans le procédé Fischer-Tropsch peut être comprise entre quelques microns et 2 millimètres. Typiquement, pour une mise en œuvre en réacteur triphasique « slurry » (en colonne à bulles), le catalyseur est finement divisé et se trouve sous forme de particules. La taille des particules de catalyseur sera comprise entre 10 et 500 micromètres (µm), de manière préférée entre 10 et 300 µm et de manière très préférée entre 20 et 150 µm, et de manière encore plus préférée entre 20 et 120 µm.

Pour illustrer l'invention et pour permettre à l'homme du métier de l'exécuter, nous présentons ci-après différents modes de réalisation du procédé de préparation d'un catalyseur utilisé pour la synthèse Fischer-Tropsch ; cependant cela ne saurait limiter la portée de l'invention.

### Exemple 1 : Préparation des catalyseurs A et B (comparatifs) et du catalyseur C (selon l'invention)

### Catalyseur A (non-conforme): Catalyseur 25 % Co sur silice-alumine à 5 % SiO2 et 5 % Co sous forme aluminate (spinelle)

Sur un support commercial Siralox® 5 (Sasol Germany), contenant 5% poids de Si02, on imprègne une solution de nitrate de cobalt, puis le solide est séché en étuve pendant 12 h à 120°C, et calciné dans un réacteur tubulaire en lit fixe à 800°C pendant 2 heures. Cette calcination à haute température permet de former la phase spinelle aluminate de cobalt 5 % poids de cobalt). Sur ce support stabilisé par du silicium et par du cobalt sous forme de spinelle, on imprègne une solution de nitrate de cobalt. Le solide obtenu est ensuite séché en étuve pendant 12 h, puis calciné sous air dans un réacteur tubulaire en lit fixe à 420°C pendant 2 heures. Il contient 15,0 % poids de cobalt. Ce solide intermédiaire subit une nouvelle imprégnation par une solution de nitrate de cobalt, puis un séchage et une calcination identiques à l'étape précédente. On obtient en deux étapes de préparation le catalyseur final A qui contient 25,0 % poids de cobalt (la teneur en Co présent dans la phase spinelle étant comprise) et une teneur en cobalt réductible de 20,0 % poids.

### Catalyseur B (non-conforme): Catalyseur 25 % Co + 0.5 % B sur silice-alumine à 5 % SiO₂ et 5 % Co sous forme aluminate (spinelle)

Sur un support commercial Siralox® 5 (Sasol Germany), contenant 5% poids de SiO₂, on imprègne une solution de nitrate de cobalt, puis le solide est séché en étuve pendant 12 h à 120°C, et calciné dans un réacteur tubulaire en lit fixe à 800°C pendant 2 heures. Cette calcination à haute température permet de former la phase spinelle aluminate de cobalt (5% poids de cobalt). Sur ce support stabilisé par du silicium et par du cobalt sous forme de spinelle, on imprègne une solution contenant du nitrate de cobalt et de l'acide borique. Le solide obtenu est ensuite séché en étuve pendant 12 h, puis calciné sous air dans un réacteur tubulaire en lit fixe à 420°C pendant 2 heures. Il contient 15,0 % poids de cobalt et 0,27 % poids de bore. Ce solide intermédiaire subit une nouvelle imprégnation par une solution de nitrate de cobalt et d'acide borique, puis un séchage et une calcination identiques à l'étape précédente. On obtient en deux étapes de préparation le catalyseur final B qui contient 25,0% poids de cobalt et 0,5% poids de bore (la teneur en Co présent dans la phase spinelle étant comprise) et une teneur en cobalt réductible de 20,0 % poids.

### Catalyseur C (selon l'invention): Catalyseur 25 % Co + 0.2 % B sur silice-alumine à 5 % SiO₂ et 5 % Co sous forme aluminate (spinelle)

Sur un support commercial Siralox® 5 (Sasol Germany), contenant 5 % poids de SiO₂, on imprègne une solution de nitrate de cobalt, puis le solide est séché en étuve pendant 12 h à 120°C, et calciné dans un réacteur tubulaire en lit fixe à 800°C pendant 2 heures. Cette calcination à haute température permet de former la phase spinelle aluminate de cobalt 5% poids de cobalt). Sur ce support stabilisé par du silicium et par du cobalt sous forme de spinelle, on imprègne une solution contenant du nitrate de cobalt et de l'acide borique. Le solide obtenu est ensuite séché en étuve pendant 12 h, puis calciné sous air dans un réacteur tubulaire en lit fixe à 420°C pendant 2 heures. Il contient 15 % poids de cobalt et 0,11 % poids de bore. Ce solide intermédiaire subit une nouvelle imprégnation par une solution de nitrate de cobalt et d'acide borique, puis un séchage et une calcination identiques à l'étape précédente. On obtient en deux étapes de préparation le catalyseur final C qui contient 25,0 % poids de cobalt et 0,2 % poids de bore (la teneur en Co présent dans la phase spinelle étant comprise) et une teneur en cobalt réductible de 20,0 % poids.

### Exemple 2 : Performances catalytiques en procédé Fischer-Tropsch des catalyseurs A à C

Les catalyseurs A à C, avant d'être successivement testés en conversion du gaz de synthèse, sont réduits *ex situ* sous un flux d'hydrogène pur à 400°C pendant 16 heures en réacteur tubulaire. Une fois le catalyseur réduit, il est déchargé sous atmosphère d'argon et enrobé par de la cire Sasolwax® pour être stocké à l'abri de l'air avant test. La réaction de synthèse Fischer-Tropsch est opérée dans un réacteur de type slurry et fonctionnant en continu et opérant avec une concentration de 10% (vol) de catalyseur en phase slurry. Chacun des catalyseurs se trouve sous forme de poudre de diamètre compris entre environ 30 et 170 microns.

Les conditions de test sont les suivantes :
Température = 220°C
Pression totale = 2 MPa
Rapport molaire H₂/CO = 2

La conversion du CO est maintenue entre 45 et 50% pendant toute la durée du test.

Les conditions de test sont ajustées de façon à être à iso conversion de CO quelle que soit l'activité du catalyseur.

Les résultats ont été calculés pour les catalyseurs A à C par rapport au catalyseur A servant de référence et figurent dans le tableau 1 ci-dessous. Les sélectivités alpha paraffines sont aussi données ainsi que la sélectivité en méthane.

La mesure de la sélectivité en alpha paraffine se fait via une analyse par chromatographie en phase gazeuse des effluents de la réaction, dosage des paraffines et calcul de la pente de la courbe log mol (%) = f(nombre de carbone) qui correspond au coefficient alpha.

**Tableau 1**

| Cat. | Formulation cible (% en poids par rapport au poids total du catalyseur) | Activité relative après 300 h de test sous charge syngas | Sélectivité de formation du méthane (%) | Sélectivité α des paraffines longues |
|---|---|---|---|---|
| A | 25% Co | 100 (base) | 10 | 0,894 |
| B | 25% Co + 0,5% B | 104 | 10,5 | 0,892 |
| C | 25% Co + 0,2% B | 145 | 10 | 0,896 |

Les résultats du tableau 1 montrent les performances catalytiques des catalyseurs A à C ; il apparaît que le catalyseur C selon l'invention présente des gains significatifs en activité par rapport aux catalyseurs comparatifs tout en gardant une sélectivité équivalente au catalyseur A.

## Revendications

1. Catalyseur contenant une phase active comprenant au moins un métal du groupe VIIIB choisi parmi le cobalt, le nickel, le ruthénium et le fer déposé sur un support contenant de la silice, de l'alumine, et au moins une spinelle simple MAl₂O₄ ou mixte MₓM'₍₁₋ₓ₎Al₂O₄ partielle ou non, où M et M' sont des métaux distincts choisis dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn) et où x est compris entre 0 et 1, les valeurs 0 et 1 étant elles-mêmes exclues,
**caractérisé en ce que** ladite phase active comprend en outre du bore, la teneur en bore étant comprise entre 0,02 % et 0,2 % en poids par rapport au poids total du catalyseur.

2. Catalyseur selon la revendication 1, **caractérisé en ce que** ledit support est une silice-alumine dans laquelle la spinelle est incluse.

3. Catalyseur selon les revendications 1 ou 2, dans lequel la teneur en spinelle est entre 3 et 50 % poids par rapport au poids du support.

4. Catalyseur selon l'une quelconque des revendications 1 à 3, dans lequel la teneur en métal M ou M' est comprise entre 1 et 20 % poids par rapport au poids du support.

5. Catalyseur selon l'une quelconque des revendications 1 à 4, dans lequel M est le cobalt dans le cas d'une spinelle simple, et M est le cobalt et M' est le magnésium ou le zinc dans le cas d'une spinelle mixte.

6. Catalyseur selon l'une quelconque des revendications 1 à 5, dans lequel la teneur en métal du groupe VIIIB de la phase active est comprise entre 0,01 et 60 % poids par rapport au poids du catalyseur.

7. Procédé de préparation d'un catalyseur selon l'une quelconque des revendications 1 à 6, lequel procédé comprenant au moins une étape dans laquelle on imprègne ledit support contenant de la silice, de l'alumine et la spinelle par une solution aqueuse ou organique comprenant au moins un sel de métal du groupe VIIIB choisi parmi le cobalt, le nickel, le ruthénium et le fer, et au moins un précurseur de bore.

8. Procédé selon la revendication 7, comprenant les étapes suivantes :
a) on fournit un support contenant de la silice et de l'alumine ;
b) on imprègne ledit support par une solution aqueuse ou organique comprenant au moins un sel de métal M ou M' choisi dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le potassium (K), le fer (Fe) et le manganèse (Mn), puis on sèche et on calcine à une température entre 700 et 1200°C, de manière à obtenir une spinelle simple MAl₂O₄ ou mixte MₓM'₍₁₋ₓ₎Al₂O₄ partielle ou non, où M et M' sont des métaux distincts et où x est compris entre 0 et 1, les valeurs 0 et 1 étant elles-mêmes exclues ;
c) on imprègne une première fois ledit support contenant de la silice, de l'alumine et la spinelle par une solution aqueuse ou organique comprenant au moins un sel de métal du groupe VIIIB choisi parmi le cobalt, le nickel, le ruthénium et le fer ;
d) on imprègne une deuxième fois ledit support contenant de la silice, de l'alumine et la spinelle par une solution aqueuse ou organique comprenant au moins un sel de métal du groupe VIIIB choisi parmi le cobalt, le nickel, le ruthénium et le fer ;
procédé dans lequel ladite solution aqueuse ou organique imprégnée à l'étape c) et/ou d) comprend en outre le précurseur de bore.

9. Procédé selon la revendication 8, dans lequel le précurseur de bore est introduite uniquement dans la solution aqueuse ou organique approvisionné à l'étape d).

10. Procédé selon la revendication 8 ou 9, dans lequel la teneur la teneur du ou des métaux du groupe VIIIB approvisionnée à l'étape c) est comprise entre 0,005 et 30 % poids par rapport au poids du catalyseur.

11. Procédé selon l'une quelconque des revendications 8 à 10, dans lequel la teneur du ou des métaux du groupe VIIIB approvisionnée à l'étape d) est comprise entre 0,005 et 30 % en poids par rapport au poids total du catalyseur.

12. Procédé selon l'une quelconque des revendications 7 à 11, dans lequel le précurseur de bore est l'acide borique.

13. Procédé selon l'une quelconque des revendications 8 à 12, dans lequel on sèche le catalyseur obtenu à l'étape d) à une température comprise entre 30 minutes et 3 heures, puis on calcine ledit catalyseur séché sous atmosphère oxydante à une température comprise entre 320°C et 460°C.

14. Procédé selon la revendication 13, dans lequel on réduit le catalyseur séché et calciné à une température comprise entre 200°C et 500°C.

15. Procédé Fischer-Tropsch de synthèse d'hydrocarbures dans lequel le catalyseur selon l'une quelconque des revendications 1 à 6 ou préparé selon l'une quelconque des revendications 7 à 14 est mis en contact avec une charge comprenant du gaz de synthèse sous une pression totale comprise entre 0,1 et 15 MPa, sous une température comprise entre 150 et 350°C, et à une vitesse volumique horaire comprise entre 100 et 20000 volumes de gaz de synthèse par volume de catalyseur et par heure avec un rapport molaire H₂/CO du gaz de synthèse entre 0,5 et 4.

## Patentansprüche

1. Katalysator, enthaltend eine aktive Phase, umfassend mindestens ein Metall der Gruppe VIIIB, ausgewählt aus Kobalt, Nickel, Ruthenium und Eisen, abgeschieden auf einem Träger, enthaltend Siliziumdioxid, Aluminiumoxid, und mindestens einen einfachen Spinell MAl₂O₄ oder gemischten Spinell MₓM'₍₁₋ₓ₎Al₂O₄, partiell oder nicht, wobei M und M' verschiedene Metalle sind, ausgewählt aus der Gruppe bestehend aus Magnesium (Mg), Kupfer (Cu), Kobalt (Co), Nickel (Ni), Zinn (Sn), Zink (Zn), Lithium (Li), Calcium (Ca), Cäsium (Cs), Natrium (Na), Kalium (K), Eisen (Fe) und Mangan (Mn) und wobei x im Bereich zwischen 0 und 1 liegt, wobei die Werte 0 und 1 selbst ausgeschlossen sind,
**dadurch gekennzeichnet, dass** die aktive Phase ferner Bor umfasst, wobei der Borgehalt im Bereich zwischen 0,02 Gew.-% und 0,2 Gew.-% liegt, bezogen auf das Gesamtgewicht des Katalysators.

2. Katalysator nach Anspruch 1, **dadurch gekennzeichnet, dass** der Träger ein Siliziumdioxid-Aluminiumoxid ist, in dem der Spinell enthalten ist.

3. Katalysator nach den Ansprüchen 1 oder 2, wobei der Spinellgehalt zwischen 3 und 50 Gew.-%, bezogen auf das Gewicht des Trägers, liegt.

4. Katalysator nach einem der Ansprüche 1 bis 3, wobei der Metallgehalt M oder M' im Bereich zwischen 1 und 20 Gew.-%, bezogen auf das Gewicht des Trägers, liegt.

5. Katalysator nach einem der Ansprüche 1 bis 4, wobei bei einem einfachen Spinell M Kobalt ist, und bei einem gemischten Spinell M Kobalt und M' Magnesium oder Zink ist.

6. Katalysator nach einem der Ansprüche 1 bis 5, wobei der Metallgehalt der Gruppe VIIIB der aktiven Phase im Bereich zwischen 0,01 und 60 Gew.-%, bezogen auf das Gewicht des Katalysators, liegt.

7. Verfahren zur Herstellung eines Katalysators nach einem der Ansprüche 1 bis 6, wobei das Verfahren mindestens einen Schritt umfasst, wobei der Träger, der Siliziumdioxid, Aluminiumoxid und Spinell enthält, mit einer wässrigen oder organischen Lösung, umfassend mindestens ein Metallsalz der Gruppe VIIIB, ausgewählt aus Kobalt, Nickel, Ruthenium und Eisen, und mindestens eine Borvorstufe, imprägniert wird.

8. Verfahren nach Anspruch 7, umfassend die folgenden Schritte:
a) Bereitstellen eines Trägers, der Siliziumdioxid und Aluminiumoxid enthält;
b) Imprägnieren des Trägers mit einer wässrigen oder organischen Lösung, umfassend mindestens ein Metallsalz M oder M', ausgewählt aus der Gruppe bestehend aus Magnesium (Mg), Kupfer (Cu), Kobalt (Co), Nickel (Ni), Zinn (Sn), Zink (Zn), Lithium (Li), Calcium (Ca), Cäsium (Cs), Natrium (Na), Kalium (K), Eisen (Fe) und Mangan (Mn), dann Trocknen und Kalzinieren bei einer Temperatur zwischen 700 und 1.200 °C, um einen einfachen Spinell MAl₂O₄ oder gemischten Spinell MₓM'₍₁₋ₓ₎Al₂O₄, partiell oder nicht, zu erhalten, wobei M und M' verschiedene Metalle sind, und wobei x im Bereich zwischen 0 und 1 liegt, wobei die Werte 0 und 1 selbst ausgeschlossen sind;
c) erstes Imprägnieren des Trägers, der Siliziumdioxid, Aluminiumoxid und Spinell enthält, mit einer wässrigen oder organischen Lösung, die mindestens ein Metallsalz der Gruppe VIIIB, ausgewählt aus Kobalt, Nickel, Ruthenium und Eisen, umfasst;
d) zweites Imprägnieren des Trägers, der Siliziumdioxid, Aluminiumoxid und Spinell enthält, mit einer wässrigen oder organischen Lösung, die mindestens ein Metallsalz der Gruppe VIIIB, ausgewählt aus Kobalt, Nickel, Ruthenium und Eisen, umfasst;
ein Verfahren, wobei die in Schritt c) und/oder d) imprägnierte wässrige oder organische Lösung ferner die Borvorstufe umfasst.

9. Verfahren nach Anspruch 8, wobei die Borvorstufe nur in die in Schritt d) zugeführte wässrige oder organische Lösung eingebracht wird.

10. Verfahren nach Anspruch 8 oder 9, wobei der Gehalt des in Schritt c) zugeführten Metalls der Gruppe VIIIB im Bereich zwischen 0,005 und 30 Gew.-%, bezogen auf das Gewicht des Katalysators, liegt.

11. Verfahren nach einem der Ansprüche 8 bis 10, wobei der Gehalt des in Schritt d) zugeführten Metalls der Gruppe VIIIB im Bereich zwischen 0,005 und 30 Gew.-%, bezogen auf das Gesamtgewicht des Katalysators, liegt.

12. Verfahren nach einem der Ansprüche 7 bis 11, wobei die Borvorstufe Borsäure ist.

13. Verfahren nach einem der Ansprüche 8 bis 12, wobei der in Schritt d) erhaltene Katalysator bei einer Temperatur im Bereich zwischen 30 Minuten und 3 Stunden getrocknet wird, der getrocknete Katalysator dann unter einer oxidierenden Atmosphäre bei einer Temperatur im Bereich zwischen 320 °C und 460 °C kalziniert wird.

14. Verfahren nach Anspruch 13, wobei der getrocknete und kalzinierte Katalysator bei einer Temperatur im Bereich zwischen 200 °C und 500 °C reduziert wird.

15. Fischer-Tropsch-Verfahren zur Synthese von Kohlenwasserstoffen, wobei der Katalysator nach einem der Ansprüche 1 bis 6 oder hergestellt nach einem der Ansprüche 7 bis 14 mit einem Einsatzmaterial, umfassend Synthesegas, unter einem Gesamtdruck im Bereich zwischen 0,1 und 15 MPa, unter einer Temperatur im Bereich zwischen 150 und 350 °C und mit einer Raumgeschwindigkeit pro Stunde im Bereich zwischen 100 und 20.000 Volumenteile Synthesegas pro Volumenteil Katalysator und pro Stunde mit einem Molverhältnis H₂/CO des Synthesegases zwischen 0,5 und 4 in Kontakt gebracht wird.

## Claims

1. A catalyst containing an active phase comprising at least one metal of group VIIIB selected from cobalt, nickel, ruthenium and iron deposited on a support containing silica, alumina and at least one simple spinel MAl₂O₄ or mixed spinel MₓM'₍₁₋ₓ₎Al₂O₄ which is or is not partial, wherein M and M' are separate metals selected from the group formed by magnesium (Mg), copper (Cu), cobalt (Co), nickel (Ni), tin (Sn), zinc (Zn), lithium (Li), calcium (Ca), caesium (Cs), sodium (Na), potassium (K), iron (Fe) and manganese (Mn), and wherein x is between 0 and 1, the values 0 and 1 being themselves excluded,
**characterised in that** said active phase further comprises boron, the boron content being between 0.02% and 0.2% by weight with respect to the total weight of the catalyst.

2. A catalyst according to claim 1 **characterised in that** said support is a silica-alumina in which the spinel is included.

3. A catalyst according to claims 1 or 2 in which the spinel content is between 3 and 50% by weight with respect to the weight of the support.

4. A catalyst according to any one of claims 1 to 3 in which the metal M or M' content is between 1 and 20% by weight with respect to the weight of the support.

5. A catalyst according to any one of claims 1 to 4 in which M is cobalt in the case of a simple spinel and M is cobalt and M' is magnesium or zinc in the case of a mixed spinel.

6. A catalyst according to any one of claims 1 to 5 in which the content of metal of group VIIIB of the active phase is between 0.01 and 60% by weight with respect to the weight of the catalyst.

7. A process for the preparation of a catalyst according to any one of claims 1 to 6, which process comprises at least one step in which said support containing silica, alumina and spinel is impregnated with an aqueous or organic solution comprising at least one metal salt of group VIIIB selected from cobalt, nickel, ruthenium and iron, and at least one boron precursor.

8. A process according to claim 7 comprising the following steps:
a) a support containing silica and alumina is provided;
b) said support is impregnated with an aqueous or organic solution comprising at least one salt of metal M or M' selected from the group formed by magnesium (Mg), copper (Cu), cobalt (Co), nickel (Ni), tin (Sn), zinc (Zn), lithium (Li), calcium (Ca), caesium (Cs), sodium (Na), potassium (K), iron (Fe) and manganese (Mn), it is then dried and calcined at a temperature between 700 and 1200°C so as to obtain a simple spinel MAl₂O₄ or mixed spinel MₓM'₍₁₋ₓ₎Al₂O₄ which is or is not partial, wherein M and M' are separate metals and x is between 0 and 1, the values 0 and 1 being themselves excluded;
c) said support containing silica, alumina and spinel is impregnated a first time with an aqueous or organic solution comprising at least one salt of metal of group VIIIB selected from cobalt, nickel, ruthenium and iron;
d) said support containing silica, alumina and spinel is impregnated a second time with an aqueous or organic solution comprising at least one salt of metal of group VIIIB selected from cobalt, nickel, ruthenium and iron;
in which process said aqueous or organic solution impregnated in step c) and/or d) further comprises the boron precursor.

9. A process according to claim 8 in which the boron precursor is introduced solely in the aqueous or organic solution supplied in step d).

10. A process according to claim 8 or claim 9 in which the content of the metal or metals of group VIIIB supplied in step c) is between 0.005 and 30% by weight with respect to the weight of the catalyst.

11. A process according to any one of claims 8 to 10 in which the content of the metal or metals of group VIIIB supplied in step d) is between 0.005 and 30% by weight with respect to the total weight of the catalyst.

12. A process according to any one of claims 7 to 11 in which the boron precursor is boric acid.

13. A process according to any one of claims 8 to 12 in which the catalyst obtained in step d) is dried at a temperature of between 30 minutes and 3 hours and then said dried catalyst is calcined in an oxidising atmosphere at a temperature of between 320°C and 460°C.

14. A process according to claim 13 in which the dried and calcined catalyst is reduced at a temperature of between 200°C and 500°C.

15. A Fischer-Tropsch process for the synthesis of hydrocarbons in which the catalyst according to any one of claims 1 to 6 or prepared according to any one of claims 7 to 14 is brought into contact with a feedstock comprising synthesis gas under a total pressure of between 0.1 and 15 MPa at a temperature of between 150 and 350°C and at an hourly space velocity of between 100 and 20000 volumes of synthesis gas per volume of catalyst and per hour with a H₂/CO molar ratio of the synthesis gas of between 0.5 and 4.
